(19) Europäisches Patentamt
European Patent Office
Office européen des brevets

(11) Veröffentlichungsnummer: **0 013 955**
**B1**

(12) # EUROPÄISCHE PATENTSCHRIFT

(45) Veröffentlichungstag der Patentschrift: **01.06.83**

(21) Anmeldenummer: **80100262.7**

(22) Anmeldetag: **21.01.80**

(51) Int. Cl.³: **C 07 D 307/935,**
A 61 K 31/557

(54) **Neue Analoga von Prostacyclin, deren Zwischenprodukte, Verfahren zu ihrer Herstellung sowie sie enthaltende Arzneimittel.**

(30) Priorität: **25.01.79 DE 2902809**

(43) Veröffentlichungstag der Anmeldung:
**06.08.80 Patentblatt 80/16**

(45) Bekanntmachung des Hinweises auf die Patenterteilung:
**01.06.83 Patentblatt 83/22**

(84) Benannte Vertragsstaaten:
**AT BE CH DE FR GB IT NL SE**

(56) Entgegenhaltungen:
**DE - A - 2 808 006**
**DE - A - 2 809 452**
**FR - A - 2 351 112**

**TETRAHEDRON LETTERS, Nr. 30, Juli 1977, Seiten 2627—2628 Pergamon Press, G.B. I. TOMOSKOZI et al.: "A simple synthesis of PGI21"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt/Main 80 (DE)**

(72) Erfinder: **Bartmann, Wilhelm, Dr.**
**Am Dachsbau 5**
**D-6232 Bad Soden am Taunus (DE)**
Erfinder: **Konz, Elmar, Dr.**
**Brüningstrasse 9**
**D-6233 Kelkheim (Taunus) (DE)**
Erfinder: **Lerch, Ulrich, Dr.**
**Schwalbenweg 19**
**D-6238 Hofheim am Taunus (DE)**
Erfinder: **Schölkens, Bernward, Dr.**
**Am Fliedergarten 1**
**D-6233 Kelkheim (Taunus) (DE)**

(56) Entgegenhaltungen:
**JOURNAL OF THE AMERICAN CHEMICAL SOCIETY, Band 99, Nr. 6, 16. März 1977, Seiten 2006—2008 E. J. COREY et al.: "Synthesis of Vane's Prostaglandin X, 6,9alfa-Oxido-9alfa,15alfa-dihydroxyprosta-(Z)5, (E) 13-dienoic Acid"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 013 955 B1**

(56) Entgegenhaltungen:

**TETRAHEDRON LETTERS, Nr. 40, Oktober 1977, Seiten 3529—3532 Pergamon Press, G.B. E. J. COREY et al.: "Synthesis of 6,9alfa-oxido-11alfa,15alfa-dihydroxyprosta-(E)5,(E) 13-dienoic acid, an isomer of PGI2 (Vane's PGX)"**

# Neue Analoga von Prostacyclin, deren Zwischenprodukte, Verfahren zu ihrer Herstellung sowie sie enthaltende Arzneimittel

Prostacyclin oder $PGI_2$, ein kürzlich isolierter Naturstoff aus der Familie der Prostaglandine, zeichnet sich durch seine stark ausgeprägten thrombocytenaggregationshemmenden Eigenschaften aus (The Lancet *1977*, 18). Außerdem vermag $PGI_2$ einige Blutgefäße, z.B. Coronararterien zu relaxieren (Prostaglandins *13*, 3, 1977), so daß es zur Therapie und Prophylaxe von Thrombosen und Infarkten Verwendung finden könnte.

Gegenstand der vorliegenden Erfindung sind neue Analoga der allgemeinen Formel I

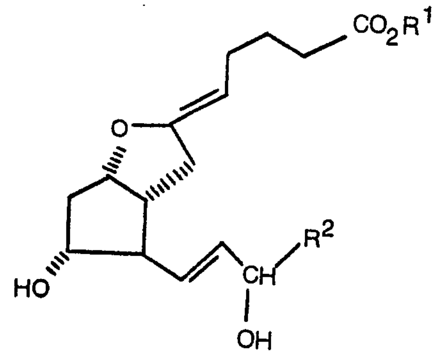

I

die eine spezifischere Wirkung und/oder eine längere Wirkungsdauer als $PGI_2$ besitzen und in welcher bedeuten:

$R^1$ einen geradkettigen oder verzweigten Alkylrest mit bis zu 6 Kohlenstoffatomen,
$R^2$ einen Cycloalkylrest mit 5 bis 7 Kohlenstoffatomen oder einen geradkettigen oder verzweigten Alkylrest mit bis zu 5 Kohlenstoffatomen der seinerseits substituiert ist mit einem $\alpha$- oder $\beta$-Furylrest oder einem $\alpha$- oder $\beta$-Oxythienylrest.

Gegenstand der Erfindung ist außerdem ein Verfahren zur Herstellung der Prostacyclinderivate der Formel I, dadurch gekennzeichnet, daß man

a) eine Verbindung der Formel II

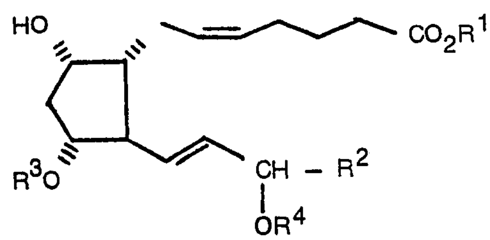

II

worin $R^1$ und $R^2$ die zur Formel I angegebenen Bedeutungen haben und $R^3$ und $R^4$ gleich oder verschieden sein können und Wasserstoff oder eine leicht abspaltbare Schutzgruppe bedeuten, in Gegenwart eines geeigneten elektrophilen Reagenz cyclisiert zu einer Verbindung der Formel III

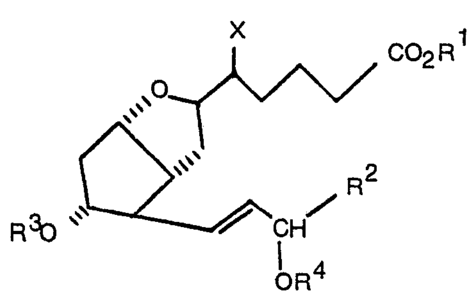

III

worin $R^1$ und $R^2$ die zur Formel I, $R^3$ und $R^4$ die zur Formel II angegebenen Bedeutungen haben und X Chlor, Brom oder Jod bedeutet,

$a_1$) gegebenenfalls in einer Verbindung der Formel III sofern $R^3$ und/oder $R^4$ eine Schutzgruppe bedeutet, diese abspaltet, wobei eine Verbindung der Formel III erhalten wird, in welcher $R^1$ und $R^2$ die zur Formel I und X die zur Formel III angegebenen Bedeutungen haben und $R^3$ und $R^4$ Wasserstoff bedeuten,

b) aus einer Verbindung der Formel III HX abspaltet, wobei eine Verbindung der Formel IV

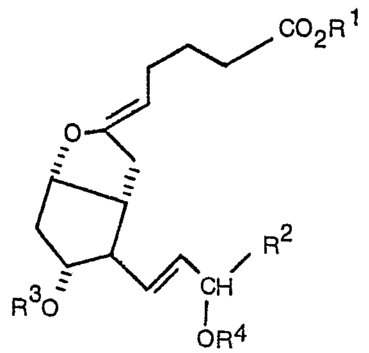

IV

erhalten wird, worin $R^1$ und $R^2$ die zur Formel I und $R^3$ und $R^4$ die zur Formel II angegebenen Bedeutungen haben

c) sofern in einer Verbindung der Formel IV $R^3$ und/oder $R^4$ nicht Wasserstoff, sondern eine Schutzgruppe bedeutet, diese unter geeigneten neutralen oder alkalischen Bedingungen abspaltet, wobei eine Verbindung der Formel I gebildet wird, worin $R^1$ und $R^2$ die zur Formel I angegebenen Bedeutungen haben,

$c_1$) gegebenenfalls aus einer Verbindung der Formel III, sofern $R^3$ und/oder $R^4$ nicht Wasserstoff, sondern eine Schutzgruppe bedeutet, diese gleichzeitig mit HX abspaltet, wobei eine Verbindung der Formel I gebildet wird, worin $R^1$ und $R^2$ die zur Formel I angegebenen Bedeutungen haben,

d) gegebenenfalls eine Verbindung der Formel I, worin $R^1$ Wasserstoff oder ein Kation bedeutet und $R^2$ die zur Formel I angegebenen Bedeutungen haben, zu einer Verbindung der Formel I verestert, worin $R^1$ ein Alkylrest mit der zur Formel I angegebenen Bedeutung ist.

Unter den Substituenten für $R^2$ sind die im folgenden aufgeführten besonders bevorzugt: 3-Thienyloxymethyl, 2-Thienyloxymethyl, 3-Furyl-2-äthyl, 2-Furyl-2-äthyl, Cyclopentyl, Cyclohexyl, Cycloheptyl.

Verbindungen der Formel I, worin $R^2$ einen gegebenenfalls durch Fluor substituierten Alkylrest, einen Alkenylrest oder einen Phenoxymethylrest darstellt, sind bereits bekannt (Tetrahedron Letters, Nr. 30, Juli 1977, S. 2627; Tetrahedron Letters Nr. 40, Okt. 1977, S. 3529; J. Am. Chem. Soc. *99*, März 1977, S. 2007; DE—OS 2 809 452, FR - A - 2 351 112). Die Verbindungen gemäß der vorliegenden Erfindung zeigen jedoch wie aus der Tabelle auf Seite 9 ersichtlich, eine überraschend starke Steigerung der blutdrucksenkenden Wirkung.

Für die Herstellung der im erfindungsgemäßen Verfahren als Ausgangsmaterial verwendeten Prostaglandinderivate der allgemeinen Formel II kann man analog den Verfahren arbeiten, wie sie z.B. in JACS 91, 5675 (1969), Tetrahedron Letters, 1970, 311, NL 7 206 361, NL 7 209 758 bzw. in DE—OS 2 524 955 und DE—OS 2 742 407 (HOE 77/F 189) beschrieben sind.

Für die Cyclisierung von Verbindungen der Formel II zu Verbindungen der Formel III eignen sich elektrophile Reagenzien, die mit $\alpha$-Hydroxyolefinen unter Ringschluß zu Tetrahydrofuranderivaten reagieren, wie z.B. Jod, Jodchlorid, $KJ_3$ oder N-Bromimide wie N-Bromsuccinimid, N-Bromcampherimid oder 1,3-Dibrom-5,5-dimethylhydantoin. Die Reaktion wird vorzugsweise in einem inerten Lösungsmittel, wie z.B. Wasser, Methylenchlorid, Chloroform, Diäthyläther, Tetrahydrofuran oder 1,2-Dimethoxyäthan durchgeführt. Es können auch heterogene oder homogene Lösungsmittelgemische verwendet werden. Die Reaktion kann bei Temperaturen zwischen −70 und +30°C durchgeführt werden, gegebenenfalls in Gegenwart eines säurebindenden Mittels wie z.B. Calciumcarbonat, Natriumcarbonat oder Natriumhydrogencarbonat. Eine bevorzugte Ausführungsform des Verfahrens besteht darin, daß man eine Verbindung der allgemeinen Formel II in Wasser bei 0—10° mit 1,2—3 Äquivalenten $KJ_3$ in Gegenwart von Natriumcarbonat unter Inertgas rührt, mit Natriumthiosulfatlösung das überschüssige $KJ_3$ reduziert und das Cyclisierungsprodukt III (X = J) mit Chloroform extrahiert. Es kann ohne besondere Reinigung weiter umgesetzt werden.

Sofern in einer Verbindung der Formel III $R^3$ und/oder $R^4$ eine Schutzgruppe bedeutet, kann diese gegebenenfalls abgespalten werden. Dies ist zweckmäßig, wenn es sich um eine Schutzgruppe handelt, die unter Säurekatalyse entfernt werden muß, wie z.B. eine Acetal- oder THP-Gruppe, da Verbindungen der Formel IV säurelabil sind. Die Schutzgruppen können unter Säurekatalyse zweckmäßigerweise in einem alkoholischen oder wäßrig/organischen Lösungsmittel abgespalten werden. Als Säure eignen sich verdünnte Mineralsäuren oder organische Säuren wie p-Toluolsulfonsäure, Oxalsäure oder Essigsäure. Handelt es sich um eine Acylschutzgruppe, so kann diese im alkalischen Milieu abgespalten werden.

Die Abspaltung von HX aus Verbindungen der Formel III unter Bildung von Verbindungen der allgemeinen Formel IV verläuft unter der Einwirkung von Basen in Gegenwart oder Abwesenheit eines Lösungsmittels.

Als Basen kommen sowohl anorganische wie organische in Betracht, wie z.B. Alkalimetallhydroxyde oder -carbonate, Alkoholate wie z.B. Natriummethylat oder Kaliumtertiärbutylat, Amine wie z.B. Triäthylamin, 4-Dimethylaminopyridin, Dicyclohexyläthylamin oder 1,4-Diazabicyclo[2,2,2]octan, Amidine wie z.B. 1,5-Diazabicyclo[3,4,0]nonen-5 (DBN) oder 1,5-Diazabicyclo[5,4,0]undecen-5 (DBU).

Sofern in einer Verbindung der Formel IV $R^3$ und/oder $R^4$ nicht Wasserstoff, sondern eine Schutzgruppe wie z.B. eine Acylgruppe bedeutet, so kann diese unter milden alkalischen Bedingungen z.B. mit Natrium- oder Kaliumcarbonat in alkoholischer oder alkoholisch-wäßriger Lösung abgespalten werden. Man arbeitet dabei bei −10 bis +30°C.

Man kann auch die Abspaltung von HX und der Schutzgruppen $R^3$ und/oder $R^4$, sofern sie Acylgruppen bedeuten, aus Verbindungen der Formel III gleichzeitig durchführen. Dazu eignet sich z.B. die Reaktion mit Alkalihydroxyden oder Metallalkoholaten in Wasser oder einem niedrig-Alkylalkohol, wie z.B. Natriummethylat in Methanol. Das Prostacyclinderivat der Formel I kann da durch direkt gewonnen werden.

Verbindungen der Formel I, worin $R^1$ Wasserstoff oder ein Kation bedeutet und $R^2$ die zur Formel I angegebenen Bedeutungen hat, lassen sich zu Verbindungen der Formel I verestern, worin $R^1$ einen Alkylrest bedeutet. Wegen der Instabilität der Enolätherstruktur im Prostacyclinmolekül kommen hierfür nur Verfahren in Frage, die rasch und unter milden Bedingungen im neutralen oder schwach sauren Milieu, oder vorteilhafterweise im alkalischen Milieu ablaufen. So kann man z.B. ein Prostacyclinderivat der Formel I ($R^1$ = H) bei Temperaturen zwischen −40 und +20° mit Diazoalkanen der Formel $R^1—N_2$ ($R^1$ = Alkyl) verestern, wobei die üblichen Lösungsmittel wie z.B. Diäthyläther, Tetrahydrofuran, Chloroform oder nieder-molekulare Alkohole wie Methanol verwendet werden können. Die resultierenden Ester können in einfacher Weise durch Eindampfen des Lösungsmittels isoliert und ggf. chromatographisch gereinigt werden.

Eine bevorzugte Veresterungsmethode besteht darin, daß man das Salz des entsprechenden Prostacyclinderivates I ($R^1$ = Kation) in Gegenwart einer Base wie z.B. eines Metallalkoholates oder Metallcarbonates in einem geeigneten Lösungsmittel mit einem Alkylierungsmittel $R^1—X$ umsetzt. Als Metallalkoholate kommen z.B. Natriummethylat, Natriumäthylat oder Kaliumtertiärbutylat in Betracht, als Carbonate eignen sich z.B. Calciumcarbonat oder Natriumhydrogencarbonat. Als geeignetes Lösungsmittel kommen Alkohole wie z.B. Methanol oder tert. Butanol, Äther wie Tetrahydrofuran oder 1,2-Dimethoxyäther und insbesondere dipolare aprotische Lösungsmittel wie Dimethylformamid, Dimethylsulfoxyd, Acetonitril oder N-Methylpyrrolidon in Betracht. In den Alkylierungsmitteln $R^1—X$ bedeutet X vorzugsweise Brom oder Jod oder einen Sulfonsäurerest.

Die Verbindungen der allgemeinen Formel II und III können als Diasteromerengemisch bezüglich der Stellung der Hydroxylgruppen am Kohlenstoffatom 15 (Prostaglandinnomenklatur), als reine $\alpha$- oder $\beta$-Isomere oder in Form von optisch aktiven Antipoden für die nachfolgenden Reaktionen eingesetzt werden. Die Trennung von Stereoisomeren bzw. Antipoden kann aber auch nach jeder folgenden Reaktionsstufe erfolgen. Das bedeutet, daß alle beschriebenen Reaktionen mit Diastereomerengemischen, reinen Diastereomeren oder optisch aktiven Antipoden durchgeführt werden können. Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Nach den erfindungsgemäßen Verfahren lassen sich außer den in den Beispielen genannten insbesondere auch die folgenden Verbindungen herstellen:

16-(2-Thienyloxy)-17,18,19,20-tetranor-$PGI_2$
17-(3-Furyl)-18,19,20-trinor-$PGI_2$
15-Cyclopentyl-16,17,18,19,20-pentanor-$PGI_2$-propylester
15-Cyclopentyl-16,17,18,19,20-pentanor-$PGI_2$

Die erfindungsgemäßen Verbindungen zeichnen sich aus durch:

1. hemmende Wirkung auf die Thrombocytenaggregation,
2. Relaxation der Gefäßwand, besonders der Coronararterien,
3. blutdrucksenkende Eigenschaften.

Sie können daher als Arzneimittel angewandt werden.

Für die blutdrucksenkende Wirkung kommen als Einheitsdosis 0,1 $\mu$g/kg—100 $\mu$g/kg, bevorzugt 5 $\mu$g/kg—100 $\mu$g/kg Körpergewicht, als Tagesdosis 0,001 mg/kg—1 mg/kg, bevorzugt 0,05 mg/kg—1 mg/kg Körpergewicht in Frage.

Für die beiden anderen Indikationen kommen die gleichen Dosen oder auch geringere Dosen in Betracht.

Pharmakologische Parameter der Verbindungen der allgemeinen Formel I ($R^1=CH_3$)

| Bsp. | R2= | Blutdrucksenkung $\mu$/kg i.v. (Ratte) ED 25 | Dosis | % | Wirkdauer min. | Rindercoronararterie-Relaxation in % zum jeweiligen Standard 1 | 10 | 100 | 1000 ng/ml | Blutplättchen-aggregation |
|---|---|---|---|---|---|---|---|---|---|---|
| | | | 0.1 | –7 | 2.4 | | | | | |
| 2d | 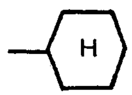 | 0.23 | 0.3 | –28 | 7.4 | 14 | 40 | 89 | | $5 \times 10^{-7}$ |
| | | | 1.0 | –61 | 18.0 | | | | | |
| | | | 0.1 | –13 | 2.4 | | | | | |
| 2a | $CH_2$–O 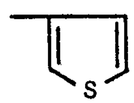 | 0.23 | 0.3 | –19 | 4.2 | 34 | 74 | 152 | | |
| | | | 1.0 | –46 | 6.4 | | | | | |
| | | | 0.1 | –6 | 1.7 | | | | | |
| 2f | –$CH_2$–$CH_2$ 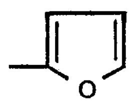 | 0.31 | 0.3 | –20 | 4.4 | 14 | 31 | 60 | | $4 \times 10^{-7}$ |
| | | | 1.0 | –49 | 11.0 | | | | | |
| Verbindungen gemäß DE–OS 2 809 452: | | | | | | | | | | |
| | –$CH_2$O 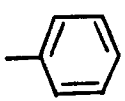 | bei 1 $\mu$g/kg i.v. leichte Senkung (15 mm Hg) | | | | 250 | 500 | 1000 | | $4 \times 10^{-6}$ |
| | | | | | | 41 | 106 | 257 | | |
| | | | 0.1 | –3 | 0.4 | | | | | |
| | 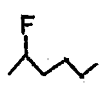 | 0.93 | 1.0 | –24 | 4.1 | 24 | 48 | 105 | | $9 \times 10^{-8}$ |
| | | | 10.0 | –52 | 8.0 | | | | | |
| | | | 100.0 | –64 | 28.8 | | | | | |

Die erfindungsgemäßen Verbindungen können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykol der auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten, sowie örtlich anwendbare Zubereitungen wie Cremes, Emulsionen, Suppositorien oder Aerosole in Frage kommen.

Die Verbindungen der Formel III und IV sind neue wertvolle Zwischenprodukte für die Herstellung von Verbindungen der Formel I.

Beispiel 1

a) (5-Brom-16(3-thienyloxy)-17,18,19,20-tetranor-PGI1 (III)

Zu 360 mg 16(3-Thienyloxy)-17,18,19,20-tetranor-$PGF_{2\alpha}$-11, 15-bis-tetrahydropyranyläther in 7 ml Tetrahydrofuran/Chloroform 1:1 gibt man unter Rühren und Argon 132 mg N-Bromsuccinimid. Nach 2 Stunden wird Chloroform und wäßrige Natriumthiosulfatlösung zugegeben und die organische Schicht nach dem Trocknen mit Magnesiumsulfat eingedampft. Den Rückstand erwärmt man unter Argon in 5 ml Eisessig/Wasser/Tetrahydrofuran 3:1:1 fünf Stunden auf 45° und verdampft danach das Lösungsmittel im Vakuum. Der Rückstand kann an etwa 20 g Silicagel säulenchromatographisch gereinigt werden. Als Elutionsmittel dient Äthylacetat/Eisessig 99:1. NMR: wie Bsp. 1c).

b) 5-Jod-16(3-thienyloxy)-17,18,19,20-tetranor-$PGI_1$-methylester (III)

200 mg 16(3-Thienyloxy)-17,18,19,20-tetranor-$PGF_{2\alpha}$-methylester (II) werden in 1,5 ml Diäthyläther gelöst und nach Zugabe von 0,3 g Kaliumhydrogencarbonat in 1 ml $H_2O$ unter Argon auf 0° gekühlt. Innerhalb von 2 Stunden werden 5,4 ml einer 2,5 %igen Lösung von Jod in Äther zugetropft und danach weitere zwei Stunden bei 0° gerührt. Nach der Zugabe von etwa 10 ml Äther wird die organische Schicht zuerst mit Natriumthiosulfatlösung, danach mit Wasser gewaschen, getrocknet und eingedampft. Das Reaktionsprodukt ist genügend rein, kann jedoch durch Chromatographie an Silicagel (Cyclohexan/Äthylacetat 2:8) von geringen Mengen an Verunreinigungen befreit werden.
Ausbeute: 250 mg, $R_f$-Wert in Essigsäureäthylester = 0.54, NMR: wie Bsp. 1c).

c) 5-Jod-16(3-thienyloxy)-17,18,19,20-tetranor-$PGI_2$-methylester (III)

200 mg 16(3-Thienyloxy)-17,18,19,20-tetranor-$PGF_{2\alpha}$-methylester (II) werden zusammen mit 50,8 mg Jod, 66 mg Kaliumjodid und 42,4 mg Natriumcarbonat in 2 ml Wasser zwei Stunden bei 5—10°C unter Inertgas gerührt. Mit Natriumthiosulfatlösung wird anschließend das überschüssige Jod entfärbt und die Reaktionsmischung mit Chloroform extrahiert. Die organische Phase wird mit Wasser gewaschen, getrocknet und eingedampft.
Ausbeute: 244 mg $R_f$-Wert in Essigsäureäthylester = 0.54

| | | | |
|---|---|---|---|
| NMR: | 5,5 — 5,7 | (m,2H) | olefin. Protonen |
| | 3.60 | (S,3H) | $O—CH_3$ |
| | 1,1 — 2,7 | (m,12H) | $—CH_2—, >CH—,$ |
| | 6,15— 7,7 | (m,3H) | Thiophen |

d) 5-Jod-15-Cyclohexyl-16,17,18,19,20-pentanor-$PGI_1$-methylester (III)

Reaktion analog Beispiel 1b) aus 15(Cyclohexyl)-16,17,18,19,20-pentanor-$PGF_{2\alpha}$-methylester (II)

| | | | |
|---|---|---|---|
| NMR: $\delta$ | 0,8 — 2,8 | (m,23H) | $—CH_2, —CH—$ |
| | 3,6 | (S,3H) | $OCH_3$ |
| | 5,5 — 5,6 | (m,2H) | olefin. Prot. |

e) 5-Jod-17(2-Furyl)-18,19,20-trinor-$PGI^1$-methylester (III)

Reaktion analog Beispiel 1c) aus 17(3-Furyl)-18,19,20-trinor-$PGF_{2\alpha}$-methylester (II)

| | | | |
|---|---|---|---|
| NMR: $\delta$ | 1,0 — 2,9 | (m,12H) | $—CH_2—, —CH—$ |
| | 3,6 | (S,3H) | $—OCH_3$ |
| | 5,5 — 5,6 | (m,2H) | olefin. Prot. |
| | 5,9 — 6,05 | | |
| u. | 6,15— 6,3 | (m,2H) | aromat. Prot. |
| | 7,2 — 7,35 | (m,1H) | " " |

Beispiel 2

a) 16(3-Thienyloxy)-17,18,19,20-trinor-$PGI_2$-methylester (I)

Unter einer Argonatmosphäre werden 300 mg 16(Thienyloxy)-5-Brom-17,18,19,20-tetranor-

$PGI_1$ (Beispiel 1a) mit 224 mg Kaliumtertiärbutylat in 10 ml tert. Butanol auf 45—50° erwärmt. Nach zweistündigem Rühren wird das Lösungsmittel im Vakuum verdampft, zum Rückstand Eiswasser und kalter Äther gegeben und die wäßrige Phase mit einer kalten Natriumdihydrogenphosphatlösung schnell auf pH 5 gebracht. Die Ätherphase wird sofort mit überschüssigem Diazomethan in kaltem Äther versetzt. Nach 30 Minuten bei —5 bis 0° reinigt man das Produkt an 15 g Kieselgel, das vorher einige Stunden in einem Gemisch von Äthylacetat/Triäthylamin 95:5 aufgeschlämmt war. Man benutzt Äthylacetat/Triäthylamin 98:2 als Elutionsmittel.
$R_f$-Wert = 0,15 (15$\alpha$-Epimeres) (in Essigester/0,5 % $N(C_2H_5)_3$)

| | | | |
|---|---|---|---|
| NMR: $\delta$ | 1,1 — 2,8 | (m,12H) | $CH_2$, CH |
| | 2,8 — 3,0 | (S,2H) | OH |
| | 3,6 | (m,3H) | $OCH_3$ |
| | 5,6 — 5,7 | (m,2H) | olefin. Prot. |
| | 6,2 — 7,2 | (m,3H) | Thiophen |

b) 15-Cyclohexyl-16,17,18,19,20-pentanor-$PGI_2$-methylester (I)
Reaktion analog Beispiel 2b aus 5-Jod-15-Cyclohexyl-16,17,18,19,20-pentanor-$PGI_1$-methylester (Beispiel 1f).

| | | | |
|---|---|---|---|
| NMR: $\delta$ | 0,8 — 2,8 | (m,23H) | $—CH_2—$, —CH— |
| | 3,65 | (S,3H) | $OCH_3$ |
| | 3,65— 4,8 | (m,4H) | —CHOH, —O—C=CH |
| | 5,5 — 5,7 | (m,2H) | olef. Prot. |

c) 17(2-Furyl)-18,19,20-trinor-$PGI_2$-methylester (I)
Reaktion analog Beispiel 2b aus 5-Jod-17(3-Furyl)-18,19,20-trinor-$PGI_1$-methylester (Beispiel 1h).

| | | | |
|---|---|---|---|
| NMR: $\delta$ | 1,0 — 2,85 | (m,12H) | $—CH_2—$, —CH— |
| | 3,6 | (S,3H) | $OCH_3$ |
| | 3,6 — 4,8 | (m,4H) | —CHOH, —O—C=CH— |
| | 5,9 — 6,05 | — | |
| u. | 6,15— 6,3 | (m,2H) | aromat. Prot. |
| | 7,2 — 7,35 | (m,1H) | aromat. Prot. |

**Patentansprüche für die Vertragsstaaten: BE CH DE FR GB IT NL SE LI**

1. Verbindungen der Formel I

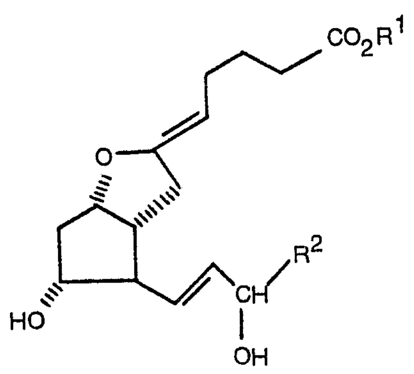

I

worin bedeuten:

$R^1$ einen geradkettigen oder verzweigten Alkylrest mit bis zu sechs Kohlenstoffatomen,
$R^2$ einen Cycloalkylrest mit 5 bis 7 Kohlenstoffatomen oder einen geradkettigen oder verzweigten Alkylrest mit bis zu 5 Kohlenstoffatomen, der seinerseits substituiert ist mit einem $\alpha$- oder $\beta$-Furylrest oder einem $\alpha$- oder $\beta$-Oxythienylrest.

2. Verfahren zur Herstellung von Verbindungen der Formel I gemäß Anspruch 1, dadurch gekennzeichnet, daß man

a) eine Verbindung der Formel II

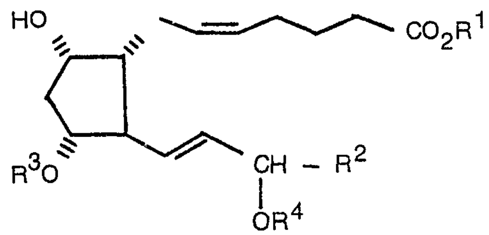

II

worin $R^1$ und $R^2$ die zur Formel I angegebenen Bedeutungen haben und $R^3$ und $R^4$ gleich oder verschieden sein können und Wasserstoff oder eine leicht abspaltbare Schutzgruppe bedeuten, in Gegenwart eines geeigneten elektrophilen Reagenz cyclisiert zu einer Verbindung der Formel III

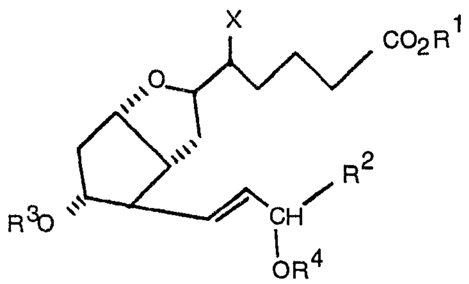

III

worin $R^1$ und $R^2$ die zur Formel I, $R^3$ und $R^4$ die zur Formel II angegebenen Bedeutungen haben und X Chlor, Brom oder Jod bedeutet,

$a_1$) gegebenenfalls in einer Verbindung der Formel III sofern $R^3$ und/oder $R^4$ eine Schutzgruppe bedeutet, diese abspaltet, wobei eine Verbindung der Formel III erhalten wird, in welcher $R^1$ und $R^2$ die zur Formel I und X die zur Formel III angegebenen Bedeutungen haben und $R^3$ und $R^4$ Wasserstoff bedeuten,

b) aus einer Verbindung der Formel III HX abspaltet, wobei eine Verbindung der Formel IV

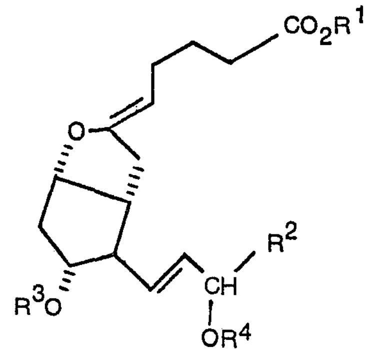

IV

erhalten wird, worin $R^1$ und $R^2$ die zur Formel I und $R^3$ und $R^4$ die zur Formel II angegebenen Bedeutungen haben,

c) sofern in einer Verbindung der Formel IV $R^3$ und/oder $R^4$ nicht Wasserstoff, sondern eine Schutzgruppe bedeutet, diese unter geeigneten neutralen oder alkalischen Bedingungen abspaltet, wobei eine Verbindung der Formel I gebildet wird, worin $R^1$ und $R^2$ die zur Formel I angegebenen Bedeutungen haben,

$c_1$) gegebenenfalls aus einer Verbindung der Formel III, sofern $R^3$ und/oder $R^4$ nicht Wasserstoff, sondern eine Schutzgruppe bedeutet, diese gleichzeitig mit HX abspaltet, wobei eine Verbindung der Formel I gebildet wird, worin $R^1$ und $R^2$ die zur Formel I angegebenen Bedeutungen haben,

d) gegebenenfalls eine Verbindung der Formel I, worin $R^1$ Wasserstoff oder ein Kation bedeutet und $R^2$ die zur Formel I angegebenen Bedeutungen haben, zu einer Verbindung der Formel I verestert, worin $R^1$ ein Alkylrest mit der zur Formel I angegebenen Bedeutung ist, und $R^2$ die zur Formel I angegebenen Bedeutungen hat.

3. Verbindungen der Formel III

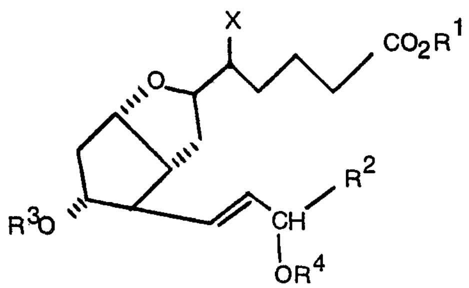

III

worin $R^1$ und $R^2$ die zur Formel I, $R^3$ und $R^4$ die zur Formel II in Anspruch 2 angegebenen Bedeutungen haben und X Chlor, Brom oder Jod bedeutet.

4. Verbindungen der Formel IV

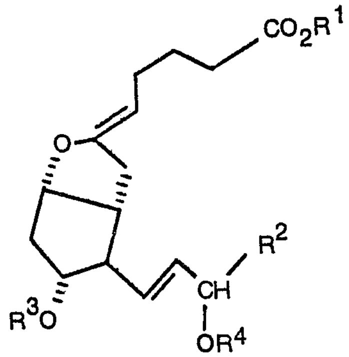

IV

worin $R^1$ und $R^2$ die zur Formel I gemäß Anspruch 1 und $R^3$ und $R^4$ die zur Formel II gemäß Anspruch 2 angegebenen Bedeutungen haben.

5. Verfahren zur Herstellung von pharmazeutischen Präparaten zur Behandlung von Thrombosen und/oder Infarkten, dadurch gekennzeichnet, daß man eine Verbindung der Formel I gemäß Anspruch 1, gegebenenfalls mit pharmazeutisch üblichen Trägerstoffen und/oder Stabilisatoren in eine therapeutisch geeignete Anwendungsform bringt.

6. Pharmazeutische Präparate zur Behandlung von Thrombosen und/oder Infarkten, gekennzeichnet durch einen Gehalt an einer Verbindung der Formel I in Anspruch 1.

7. Verbindungen der Formel I gemäß Anspruch 1 zur Verwendung als Arzneimittel.

**Patentanspruch für den Vertragsstaat: AT**

Verfahren zur Herstellung von Verbindungen der Formel I

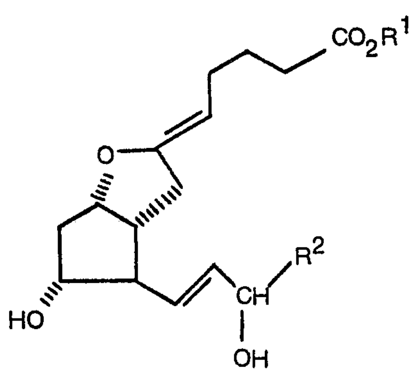

I

worin bedeuten:

$R^1$ einen geradkettigen oder verzweigten Alkylrest mit bis zu sechs Kohlenstoffatomen,

$R^2$ einen Cycloalkylrest mit 5 bis 7 Kohlenstoffatomen oder einen geradkettigen oder verzweigten Alkylrest mit bis zu 5 Kohlenstoffatomen, der seinerseits substituiert ist mit einem $\alpha$- oder $\beta$-Furylrest oder einem $\alpha$- oder $\beta$-Oxythienylrest,

dadurch gekennzeichnet, daß man

a) eine Verbindung der Formel II

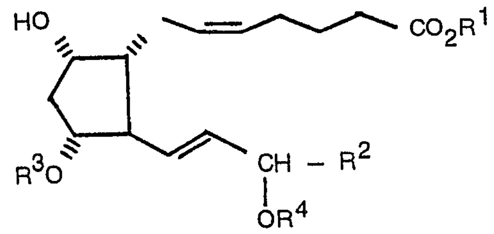

II

worin $R^1$ und $R^2$ die zur Formel I angegebenen Bedeutungen haben und $R^3$ und $R^4$ gleich oder verschieden sein können und Wasserstoff oder eine leicht abspaltbare Schutzgruppe bedeuten, in Gegenwart eines geeigneten elektrophilen Reagenz cyclisiert zu einer Verbindung der Formel III

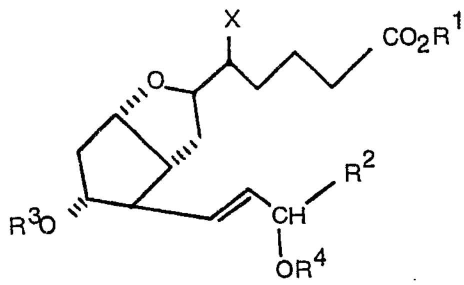

III

worin $R^1$ und $R^2$ die zur Formel I, $R^3$ und $R^4$ die zur Formel II angegebenen Bedeutungen haben und X Chlor, Brom oder Jod bedeutet,

$a_1$) gegebenenfalls in einer Verbindung der Formel III sofern $R^3$ und/oder $R^4$ eine Schutzgruppe bedeutet, diese abspaltet, wobei eine Verbindung der Formel III erhalten wird, in welcher $R^1$ und $R^2$ die zur Formel I und X die zur Formel III angegebenen Bedeutungen haben und $R^3$ und $R^4$ Wasserstoff bedeuten,

b) aus einer Verbindung der Formel III HX abspaltet, wobei eine Verbindung der Formel IV

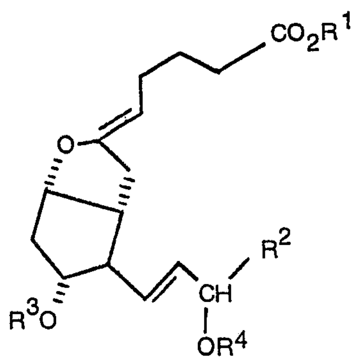

IV

erhalten wird, worin $R^1$ und $R^2$ die zur Formel I und $R^3$ und $R^4$ die zur Formel II angegebenen Bedeutungen haben,

c) sofern in einer Verbindung der Formel IV $R^3$ und/oder $R^4$ nicht Wasserstoff, sondern eine Schutzgruppe bedeutet, diese unter geeigneten neutralen oder alkalischen Bedingungen abspaltet, wobei eine Verbindung der Formel I gebildet wird, worin $R^1$ und $R^2$ die zur Formel I angegebenen Bedeutungen haben,

$c_1$) gegebenenfalls aus einer Verbindung der Formel III, sofern $R^3$ und/oder $R^4$ nicht Wasserstoff, sondern eine Schutzgruppe bedeutet, diese gleichzeitig mit HX abspaltet, wobei eine Verbindung der Formel I gebildet wird, worin $R^1$ und $R^2$ die zur Formel I angegebenen Bedeutungen haben,

d) gegebenenfalls eine Verbindung der Formel I, worin $R^1$ Wasserstoff oder ein Kation bedeutet und $R^2$ die zur Formel I angegebenen Bedeutungen haben, zu einer Verbindung der Formel I verestert, worin $R^1$ ein Alkylrest mit der zur Formel I angegebenen Bedeutung ist, und $R^2$ die zur Formel I angegebenen Bedeutungen hat.

**Claims for the Contracting States: BE CH DE FR GB IT NL SE LI**

1. Compounds of the formula I

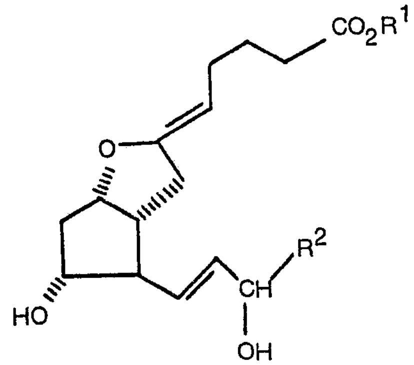

I

in which $R^1$ denotes, a straight-chain or branched alkyl radical with up to six carbon atoms, and $R^2$ denotes a cycloalkyl radical with 3 to 7 carbon atoms or a straight-chain or branched alkyl radical with up to 5 carbon atoms, which in turn is substituted by an $\alpha$- or $\beta$-furyl radical, or an $\alpha$- or $\beta$-oxythienyl radical.

2. Process for the preparation of compounds of the formula I as claimed in claim 1 which comprises

a) cyclizing a compound of the formula II

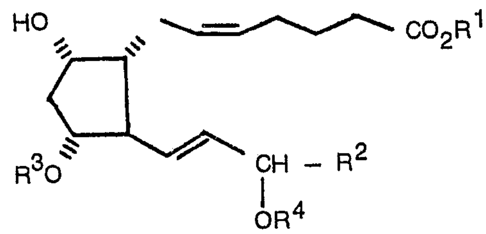

II

in which $R^1$ and $R^2$ have the meanings indicated for formula I and $R^3$ and $R^4$ can be identical or different and denote hydrogen or a protective group which can easily be split off, in the presence of a suitable electrophilic reagent to give a compound of the formula III

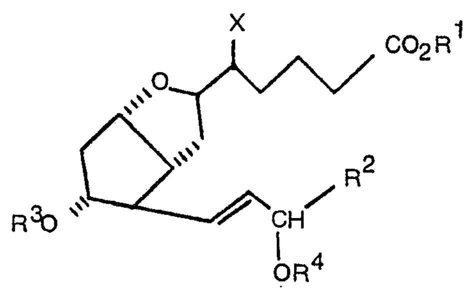

III

in which $R^1$ and $R^2$ have the meanings indicated for formula I, $R^3$ and $R^4$ have the meaning indicated for formula II and X denotes chlorine, bromine or iodine,

$a_1$) if $R^3$ and/or $R_4$ in a compound of the formula III denote a protective group, optionally splitting off this group whereupon a compound of the formula III in which $R^1$ and $R^2$ have the meanings indicated for formula I, X has the meanings indicated for formula III and $R^3$ and $R^4$ denote hydrogen is obtained.

b) splitting off HX from a compound of the formula III, whereupon a compound of the formula IV

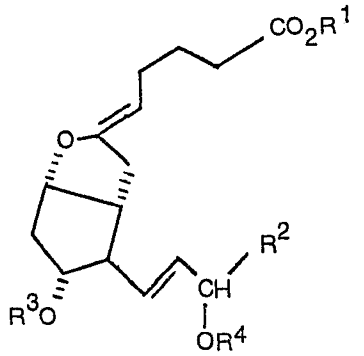

IV

in which $R^1$ and $R^2$ have the meanings indicated for formula I and $R^3$ and $R^4$ have the meanings indicated for formula II is obtained,

c) if $R^3$ and/or $R^4$ in a compound of the formula IV do not denote hydrogen but a protective group, splitting off this group under suitable neutral or alkaline conditions, whereupon a compound of the formula I in which $R^1$ and $R^2$ have the meanings indicated for formula I is formed,

$c_1$) if appropriate, if $R^3$ and/or $R^4$ do not denote hydrogen but a protective group, splitting this off from a compound of the formula III simultaneously with HX, whereupon a compound of the formula I in which $R^1$ and $R^2$ have the meanings indicated for formula I is obtained.

d) if appropriate esterifying a compound of the formula I in which $R^1$ denotes hydrogen or a cation and $R^2$ has the meanings indicated for formula I, to give a compound of the formula I in which $R^1$ is an alkyl radical with the meaning indicated for formula I and $R^2$ has the meaning indicated for formula I.

3. Compounds of the formula III

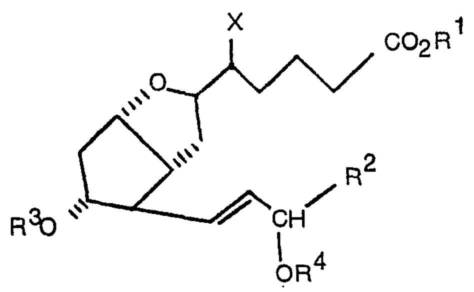

III

in which $R^1$ and $R^2$ have the meanings indicated for formula I, $R^3$ and $R^4$ have the meanings indicated for formula II in claim 2 and X denotes chlorine, bromine or iodine.

4. Compounds of the formula IV

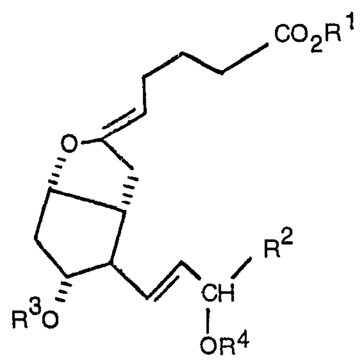

IV

in which $R^1$ and $R^2$ have the meanings indicated for formula I in claim 1 and $R^3$ and $R^4$ have the meanings indicated for formula II, in claim 2.

5. Process for the production of pharmaceutical preparations for the treatment of the thromboses and/or infarctions, which comprises converting a compound of the formula I as claimed in claim 1 into a therapeutically suitable use form, if appropriate with excipients and/or stabilisers customary in pharmaceuticals.

6. Pharmaceutical preparations for the treatment of thromboses and/or infarctions, which contain a compound of the formula I in claim 1.

7. A compound of the formula I as claimed in claim 1 for use as medicament for combating thromboses and/or infarctions, and high blood pressure.

**Claim for the Contracting State: AT**

Process for the preparation of compounds of the formula I

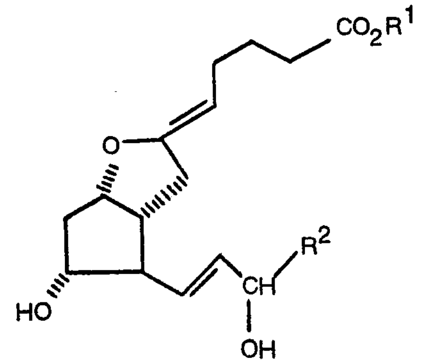

I

in which $R^1$ denotes a straight-chain or branched alkyl radical with up to six carbon atoms and $R^2$ denotes a cycloalkyl radical with 3 to 7 carbon atoms or a straight-chain or branched alkyl radical with up to 5 carbon atoms which in turn is substituted by an $\alpha$- or $\beta$-furyl radical or an $\alpha$- or $\beta$-oxythienyl radical, which comprises

a) cyclizing a compound of the formula II

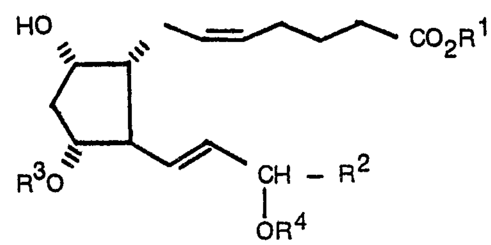

II

in which $R^1$ and $R^2$ have the meanings indicated for formula I and $R^3$ and $R^4$ can be identical or different and denote hydrogen or a protective group which can easily be split off, in the presence of a suitable electrophilic reagent to give a compound of the formula III

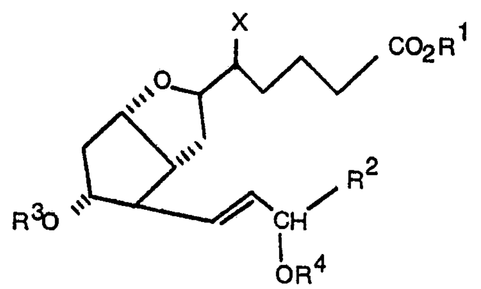

III

in which $R^1$ and $R^2$ have the meanings indicated for formula I, $R^3$ and $R^4$ have the meaning indicated for formula II and X denotes chlorine, bromine or iodine,

$a_1$) if $R^3$ and/or $R^4$ in a compound of the formula III denote a protective group, optionally splitting off this group whereupon a compound of the formula III in which $R^1$ and $R^2$ have the meanings indicated for formula I, X has the meanings indicated for formula III and $R^3$ and $R^4$ denote hydrogen is obtained.

b) splitting off HX from a compound of the formula III, whereupon a compound of the formula IV

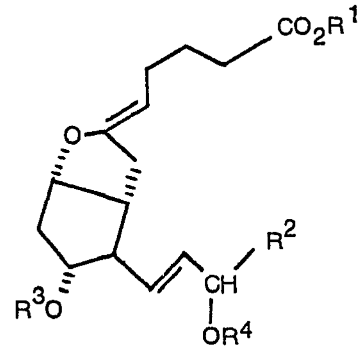

IV

in which $R^1$ and $R^2$ have the meanings indicated for formula I and $R^3$ and $R^4$ have the meanings indicated for formula II is obtained,

c) if $R^3$ and/or $R^4$ in a compound of the formula IV do not denote hydrogen but a protective group, splitting off this group under suitable neutral or alkaline conditions, whereupon a compound of the formula I in which $R^1$ and $R^2$ have the meanings indicated for formula I is formed,

$c_1$) if appropriate, if $R^3$ and/or $R^4$ do not denote hydrogen but a protective group, splitting this off from a compound of the formula III simultaneously with HX, whereupon a compound of the formula I in which $R^1$ and $R^2$ have the meanings indicated for formula I is obtained.

d) if appropriate esterifying a compound of the formula I in which $R^1$ denotes hydrogen or a cation and $R^2$ has the meanings indicated for formula I, to give a compound of the formula I in which $R^1$ is an alkyl radical with the meaning indicated for formula I and $R^2$ has the meaning indicated for formula I.

**Revendications pour les Etats contractants: BE CH DE FR GB IT NL SE LI**

1. Composés répondant à la formule I

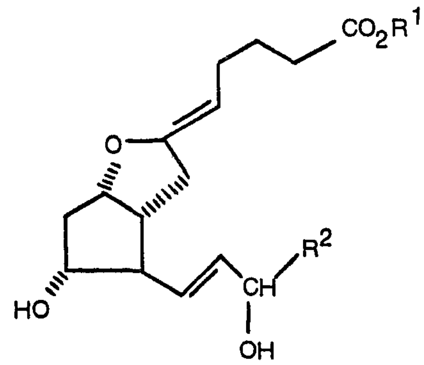

I

dans laquelle:

$R^1$ représente un groupe alcoyle à chaîne droite ou ramifiée, ayant jusqu'à 6 atomes de carbone,

$R^2$ représente un groupe cycloalcoyle ayant de 5 à 7 atomes de carbone ou un groupe alcoyle à chaîne droite ou ramifiée ayant jusqu'à 5 atomes de carbone, qui est lui-même substitué par un groupe $\alpha$- ou $\beta$-furyle ou un groupe $\alpha$- ou $\beta$-oxythiényle.

2. Procédé pour la préparation de composés de formule I selon la revendication 1, caractérisé en ce que:

a) on cyclise un composé de formule II

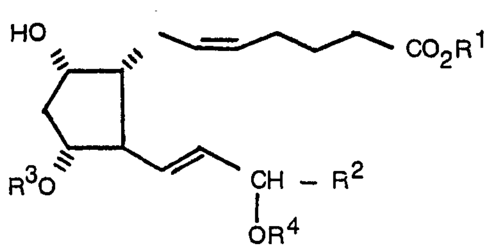

II

dans laquelle $R^1$ et $R^2$ ont les significations données pour la formule I et $R^3$ et $R^4$ peuvent être identiques ou différents et représentent l'hydrogène ou un groupe protecteur facilement éliminable, en présence d'un réactif électrophile, en un composé de formule III

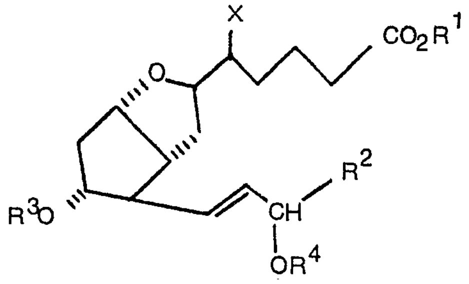

III

dans laquelle $R^1$ et $R^2$ ont les significations données pour la formule I et $R^3$ et $R^4$ ont les significations données pour la formule II, et X représente le chlore, le brome ou l'iode, $a_1$) éventuellement dans un composé de formule III, dans la mesure où $R^3$ et/ou $R^4$ représentent un groupe protecteur, on élimine ce dernier, auquel cas on obtient un composé de formule III dans laquelle $R^1$ et $R^2$ ont les significations données pour la formule I, et X a la signification donnée pour la formule III, et $R^3$ et $R^4$ représentent l'hydrogène.

b) on élimine HX d'un composé de formule III, auquel cas on obtient un composé de formule IV

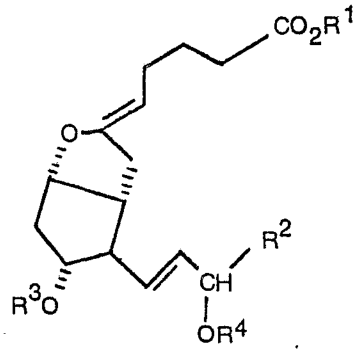

IV

dans laquelle $R^1$ et $R^2$ ont les significations données pour la formule I et $R^3$ et $R^4$ ont les significations données pour la formule II,

c) dans la mesure où dans un composé de formule IV, $R^3$ et/ou $R^4$ ne représentent pas l'hydrogène, mais un groupe protecteur, on élimine celui-ci dans des conditions neutres ou alcalines appropriées, auquel cas il se forme un composé de formule I dans laquelle $R^1$ et $R^2$ ont les significations données pour la formule I $c_1$) éventuellement, à partir d'un composé de formule III dans la mesure où $R^3$ et/ou $R^4$ ne représentent pas l'hydrogène, mais représentent un groupe protecteur, on élimine ce dernier en même temps que HX, auquel cas il se forme un composé de formule I dans laquelle $R^1$ et $R^2$ ont les significations données pour la formule I,

d) éventuellement, on estérifie un composé de formule I dans laquelle $R^1$ représente l'hydrogène ou un cation, et $R^2$ a les significations données pour la formule I, en un composé de formule I dans laquelle $R^1$ est un groupe alcoyle ayant les significations données pour la formule I et $R^2$ a la signification donnée pour la formule I.

3. Composés répondant à la formule III

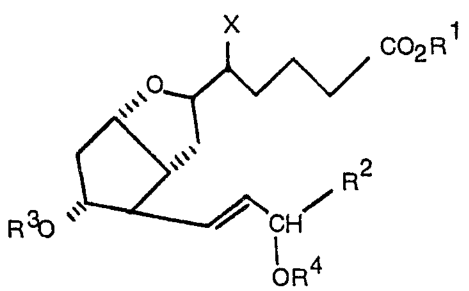

III

dans laquelle $R^1$ et $R^2$ ont les significations données pour la formule I, $R^3$ et $R^4$ ont les significations données pour la formule II dans la revendication 2 et X représente le chlore, le brome ou l'iode.

4. Composés répondant à la formule IV

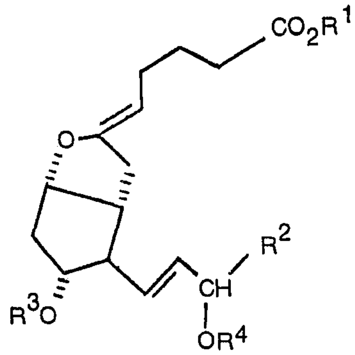

IV

dans laquelle $R^1$ et $R^2$ ont les significations données pour la formule I selon la revendication 1, et $R^3$ et $R^4$ ont les significations données pour la formule II dans la revendication 2.

5. Procédé pour la préparation de compositions pharmaceutiques pour le traitement des thromboses et/ou des infarctus, caractérisé en ce qu'on met un composé de formule I selon la revendication 1, sous une forme d'application thérapeutiquement appropriée, éventuellement avec des véhicules et/ou des stabilisants pharmaceutiquement usuels.

6. Compositions pharmaceutiques pour le traitement des thromboses et/ou des infarctus, caractérisées en ce qu'elles contiennent un composé de formule I selon la revendication 1.

7. Composés de formule I selon la revendication 1, utilisables comme médicaments.

**Revendication pour l'Etat contractant: AT**

Procédé pour la préparation de composés de formule I

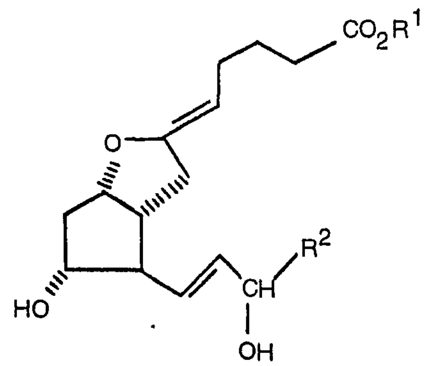

I

dans laquelle:

$R^1$ représente un groupe alcoyle à chaîne droite ou ramifiée ayant jusqu'à 6 atomes de carbone,

$R^2$ représente un groupe cycloalkyle ayant de 5 à 7 atomes de carbone, ou un groupe alcoyle à chaîne droite ou ramifiée ayant jusqu'à 5 atomes de carbone, qui est lui-même substitué par un groupe $\alpha$- ou $\beta$-furyle ou un groupe $\alpha$- ou $\beta$-oxythiényle, caractérisé en ce que:

a) on cyclise un composé de formule II

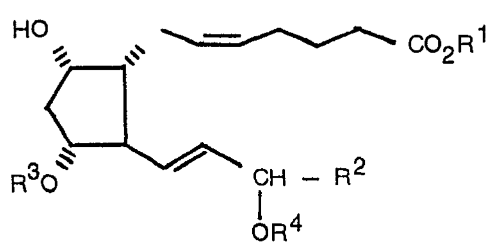

II

dans laquelle $R^1$ et $R^2$ ont les significations données pour la formule I et $R^3$ et $R^4$ peuvent être identiques ou différents et représentent l'hydrogène ou un groupe protecteur facilement éliminable, en présence d'un réactif électrophile, en un composé de formule III

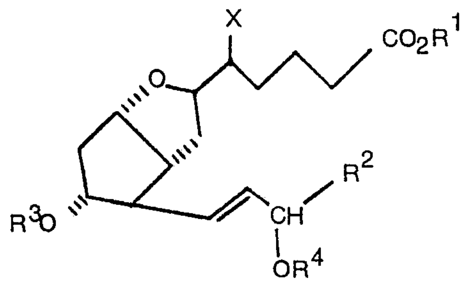

III

dans laquelle $R^1$ et $R^2$ ont les significations données pour la formule I et $R^3$ et $R^4$ ont les significations données pour la formule II, et X représente le chlore, le brome ou l'iode,

$a_1$) éventuellement dans un composé de formule III, dans la mesure où $R^3$ et/ou $R^4$ représentent un groupe protecteur, on élimine ce dernier, auquel cas on obtient un composé de formule III dans laquelle $R^1$ et $R^2$ ont les significations données pour la formule I, et X a la signification donnée pour la formule III, et $R^3$ et $R^4$ représentent l'hydrogène.

b) on élimine HX d'un composé de formule III, auquel cas on obtient un composé de formule IV

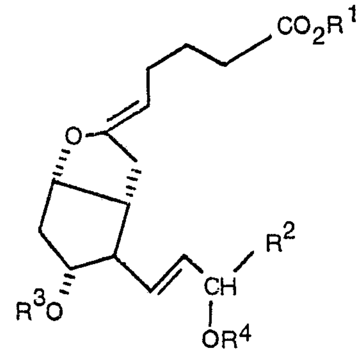

IV

dans laquelle $R^1$ et $R^2$ ont les significations données pour la formule I et $R^3$ et $R^4$ ont les significations données pour la formule II,

c) dans la mesure où dans un composé de formule IV, $R^3$ et/ou $R^4$ ne représentent pas l'hydrogène, mais un groupe protecteur, on élimine celui-ci dans des conditions neutres ou alcalines appropriées, auquel cas il se forme un composé de formule I dans laquelle $R^1$ et $R^2$ ont les significations données pour la formule I

$c_1$) éventuellement, à partir d'un composé de formule III dans la mesure où $R^3$ et/ou $R^4$ ne représentent pas l'hydrogène, mais représentent un groupe protecteur, on élimine ce dernier en même temps que HX, auquel cas il se forme un composé de formule I dans laquelle $R^1$ et $R^2$ ont les significations données pour la formule I.

d) éventuellement, on estérifie un composé de formule I dans laquelle $R^1$ représente l'hydrogène ou un cation, et $R^2$ a les significations données pour la formule I, en un composé de formule I dans laquelle $R^1$ est un groupe alcoyle ayant les significations données pour la formule I et $R^2$ a la signification donnée pour la formule I.